# EUROPEAN PATENT APPLICATION

(11) **EP 4 806 373 A2**
(43) Date of publication of application: **16.09.2026**
(21) Application number: 26186686.7
(22) Date of filing: 26.04.2022
(51) Int. Cl.: A61B 17/34

(54) **A GUIDEWIRE DELIVERY CATHETER WITH AN EXPANDABLE ANCHORING MECHANISM FOR USE IN THE CORONARY SINUS**

(30) Priority: 27.04.2021 US 202163180602 P
(62) Document number(s) of the earlier application(s) in accordance with Art. 76 EPC: 22722977.0 / 4 304 696
(71) Applicant: Edwards Lifesciences Corporation, Irvine, CA 92614 (US)
(72) Inventor: RICKERSON, Cooper Ryan, Irvine, CA, 92614 (US); COUTTEAU, Steven Charles, Iowa City, IA, 52245 (US); HALL, Bethany Jo, Portland, OR, 97229 (US); HADDAD, Jason James Raid, Irvine, CA, 92614 (US)
(74) Representative: Venner, Julia Ann

(57) **Abstract**

Mechanisms (400) are disclosed for securing a catheter in place to facilitate puncturing a hole through a vessel wall. The securing mechanisms (400) includes an expanding member (402) that expands when activated to press against the wall of the vessel (e.g., coronary sinus wall (30)). Activation of the securing mechanisms (400) occurs through removal of a restraint (e.g., a cover (401)) or by pushing or pulling a wire. The expanding member (402) may self-expand or may expand due to a spring within a mesh structure. The securing mechanisms activated by pushing or pulling a wire expands due to movement of the wire itself or due to movement of a component attached to the wire and to the anchor member.

## Description

### CROSS-REFERENCE TO RELATED APPLICATIONS

This application claims priority based on United States Provisional Patent Application Serial No. 63/180,602, filed April 27, 2021 and entitled SECURING A GUIDEWIRE DELIVERY CATHETER IN THE CORONARY SINUS USING MATERIAL OR ADVANCEMENT MECHANISMS, the complete disclosure of which is hereby incorporated by reference herein in its entirety.

### BACKGROUND

### Field

The present invention relates generally to the field of delivery devices, such as catheters, for medical procedures involving the coronary sinus.

### Description of Related Art

Heart failure is a common and potentially lethal condition affecting humans, with sub-optimal clinical outcomes often resulting in symptoms, morbidity and/or mortality, despite maximal medical treatment. In particular, "diastolic heart failure" refers to the clinical syndrome of heart failure occurring in the context of preserved left ventricular systolic function (ejection fraction) and in the absence of major valvular disease. This condition is characterized by a stiff left ventricle with decreased compliance and impaired relaxation, which leads to increased end-diastolic pressure.

Symptoms of diastolic heart failure are due, at least in a large part, to an elevation in pressure in the left atrium. Elevated left atrial pressure (LAP) is present in several abnormal heart conditions, including heart failure (HF). In addition to diastolic heart failure, a number of other medical conditions, including systolic dysfunction of the left ventricle and valve disease, can lead to elevated pressures in the left atrium. Both heart failure with preserved ejection fraction (HFpEF) and heart failure with reduced ejection fraction (HFrEF) can exhibit elevated LAP.

It may be beneficial to reduce elevated pressure in the left atrium. One way to do this is to shunt blood from the left atrium to the coronary sinus. By creating an opening between the left atrium and the coronary sinus, blood will flow from the higher pressure left atrium to the lower pressure coronary sinus. Examples of methods to shunt blood from the left atrium to the coronary sinus are disclosed in U.S. Pat. No. 9,789,294 entitled "Expandable Cardiac Shunt," the entire contents of which is incorporated by reference herein.

Using catheter-based instruments, the surgeon creates a puncture hole between the left atrium and the coronary sinus and places an expandable shunt within the puncture hole. To do this, one or more catheters are used to create the puncture hole, to deliver the expandable shunt along a guidewire, and to deploy the expandable shunt in the puncture hole. Once expanded, the shunt defines a blood flow passage that allows blood to flow from the left atrium to the coronary sinus when the LAP is elevated.

### SUMMARY

For purposes of summarizing the disclosure, certain aspects, advantages and novel features have been described herein. It is to be understood that not necessarily all such advantages may be achieved in accordance with any particular embodiment. Thus, the disclosed embodiments may be carried out in a manner that achieves or optimizes one advantage or group of advantages as taught herein without necessarily achieving other advantages as may be taught or suggested herein.

Some implementations of the present disclosure relate to a guidewire delivery catheter used to implant a shunt between the coronary sinus and the left atrium, the catheter including a securing mechanism. The securing mechanism includes a removable cover coupled to a wire or hypotube. The securing mechanism includes an expanding member activated by removal of the removable cover, the removable cover configured to be pulled by the wire or hypotube to expose the expanding member causing the expanding member to expand out of the catheter to secure a distal end of the catheter in place in the coronary sinus.

In some embodiments, the expanding member comprises a mesh structure. In some embodiments, the expanding member comprises a compliant wire material. In some embodiments, the expanding member comprises a self-expanding bubble.

In some embodiments, the expanding member comprises a spring-loaded bubble. In further embodiments, the spring is retained in a compressed state by the cover and removal of the cover allows the spring to decompress, thereby expanding the spring-loaded bubble to form a dome shape.

In some embodiments, the expanding member is configured to be retracted by re-advancing the removable cover over the expanding member. In some embodiments, activation of the expanding member causes the expanding member to press against a vessel wall to provide wall apposition for the catheter to facilitate puncturing the vessel wall.

Some implementations of the present disclosure relate to a guidewire delivery catheter used to implant a shunt between the coronary sinus and the left atrium, the catheter including a securing mechanism. The securing mechanism includes an activation member including a wire or hypotube. The securing mechanism includes an expanding member activated by the activation member, a proximal side of the expanding member coupled to a distal end of the activation member, the expanding member configured to expand out of the catheter responsive to the activation member being advanced distally, the expanding member thereby securing a distal end of the catheter in place in the coronary sinus.

In some embodiments, the expanding member comprises a mesh structure. In some embodiments, the expanding member comprises a compliant wire material. In some embodiments, the expanding member is configured to be retracted by pulling the activation member proximally. In some embodiments, a distal portion of the expanding member is fixed to the catheter. In some embodiments, activation of the expanding member causes the expanding member to press against a vessel wall to provide wall apposition for the catheter to facilitate puncturing the vessel wall. In some embodiments, the expanding member comprises a fabric covering that contains a distal portion of the activation member such that as the activation member is advanced distally, the distal portion of the activation member curves within the fabric covering to cause the fabric covering to form a dome shape.

Some implementations of the present disclosure relate to a guidewire delivery catheter used to implant a shunt between the coronary sinus and the left atrium, the catheter including a securing mechanism. The securing mechanism includes an activation member including a wire or hypotube coupled to a movable distal structure. The securing mechanism includes an expanding member activated by the activation member, the expanding member including a fixed proximal structure and the movable distal structure with pliable material forming a wall attached to the fixed proximal structure and to the movable distal structure such that pulling the activation member causes the movable distal structure to move towards the fixed proximal structure causing the wall to bulge outwards, thereby securing a distal end of the catheter in place in the coronary sinus.

In some embodiments, the wall comprises a compliant wire material. In some embodiments, the expanding member is configured to be retracted by pushing the activation member distally. In some embodiments, activation of the expanding member causes the expanding member to press against a vessel wall to provide wall apposition for the catheter to facilitate puncturing the vessel wall. In some embodiments, the activation member comprises a wire running through a hypotube, the hypotube surrounded by the fixed proximal structure.

### BRIEF DESCRIPTION OF THE DRAWINGS

Various embodiments are depicted in the accompanying drawings for illustrative purposes and should in no way be interpreted as limiting the scope of the inventions. In addition, various features of different disclosed embodiments can be combined to form additional embodiments, which are part of this disclosure. Throughout the drawings, reference numbers may be reused to indicate correspondence between reference elements. However, it should be understood that the use of similar reference numbers in connection with multiple drawings does not necessarily imply similarity between respective embodiments associated therewith. Furthermore, it should be understood that the features of the respective drawings are not necessarily drawn to scale, and the illustrated sizes thereof are presented for the purpose of illustration of inventive aspects thereof. Generally, certain of the illustrated features may be relatively smaller than as illustrated in some embodiments or configurations.
FIG. 1 illustrates several access pathways for maneuvering guidewires and catheters in and around the heart to deploy shunts.
FIG. 2 illustrates one approach method for deploying an expandable shunt, wherein a guidewire is introduced through the subclavian or jugular vein, through the superior vena cava and into the coronary sinus.
FIGS. 3A, 3B, 3C, and 3D illustrate schematic views of steps in making a puncture hole through a wall of the coronary sinus, as seen looking down on a section of the heart with the posterior aspect down.
FIGS. 4A, 4B, and 4C illustrate a first example securing mechanism that includes a self-expanding bubble and a cover.
FIGS. 5A, 5B, and 5C illustrate a second example securing mechanism that includes a spring-loaded bubble and a cover.
FIGS. 6A, 6B, and 6C illustrate a third example securing mechanism that includes a mesh structure and a wire that can be pushed to expand the mesh structure.
FIGS. 7A, 7B, and 7C illustrate a fourth example securing mechanism that includes pliable walls that expand when a wire is pulled due to proximal movement of a distal, movable structure relative to a proximal, fixed structure.

### DETAILED DESCRIPTION

The headings provided herein are for convenience only and do not necessarily affect the scope or meaning of the claimed invention.

### Overview

Symptoms of diastolic heart failure arise from elevated pressure in the left atrium, or elevated left atrial pressure (LAP). Other heart conditions may manifest elevated LAP as well. To reduce the pressure in the left atrium, a pathway can be created between the left atrium and the coronary sinus. This allows blood to flow from the higher pressure left atrium to the lower pressure coronary sinus. The pathway can be created by puncturing a hole between the left atrium and the coronary sinus. Once the hole has been created, a shunt can be placed in the hole to keep it open.

For example, catheter-based instruments can be used to create the hole and to deliver and deploy a shunt within the puncture hole. The catheter can be referred to as a guidewire delivery catheter (GDC) and can be used to puncture through the coronary sinus into the left atrium and to place a guidewire in the left atrium. To puncture through the tissue, the catheter has wall apposition to get the needle directly against the coronary sinus-left atrium wall. Prior GDCs used an "anchor balloon" (a saline-inflated balloon) to anchor the catheter when creating the puncture hole. The anchor balloon solution has the potential to burst or to slip out of position, which may harm the patient.

Accordingly, described herein are materials and mechanisms for securing the GDC in place to enable the GDC to puncture the wall from the coronary sinus to the left atrium. These materials and mechanisms serve as an alternative to the anchor balloon.

The term "catheter" is used herein according to its broad and ordinary meaning and may include any tube, sheath, steerable sheath, steerable catheter, and/or any other type of elongate tubular delivery device comprising an inner lumen configured to slidably receive instrumentation, such as for positioning within an atrium or coronary sinus, including for example delivery catheters and/or cannulas. The term "hypotube" is used herein according to its broad and ordinary meaning and may include a small-diameter tube typically made of stainless steel or nitinol that is typically used in medical devices such as catheters, stents, etc. As used herein, a wire can be used as a general term that indicates an elongated compliant structure, which may include a hypotube.

In some cases, a securing mechanism may be composed of a shape-memory alloy (e.g., Nitinol) and/or may have a pre-defined shape and/or structure. The securing mechanism may be configured to be shaped and/or compressed to fit into and/or around a catheter. In some cases, a securing mechanism may have an elongated shape to extend at least partially along the catheter in a delivery configuration and to change shape to provide wall apposition in a deployed configuration.

Some embodiments described herein provide methods and/or systems for securing a guidewire delivery catheter, or other similar delivery device, to provide wall apposition for puncturing a vessel wall. While some embodiments may be directed to securing a catheter within the coronary sinus to puncture the wall between the coronary sinus and the left atrium, the devices described herein may be applicable to other areas of the body. For example, some devices described herein may advantageously be configured for securing catheters in vessels other than the coronary sinus.

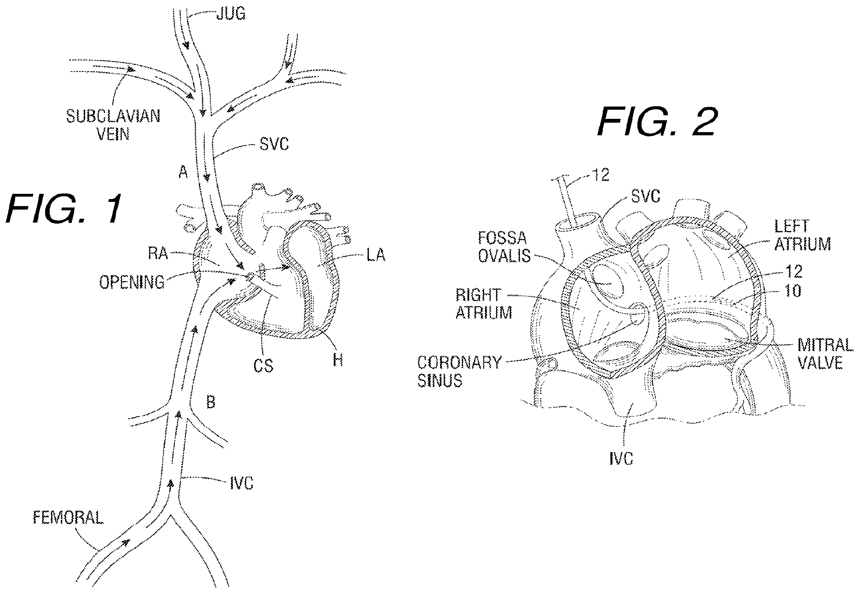

The following includes a general description of a method for delivering a guidewire delivery catheter to a targeted location in the coronary sinus. FIG. 1 illustrates several access pathways for maneuvering guidewires and catheters in and around the heart to deploy shunts. For instance, access may be from above via either the subclavian or jugular veins into the superior vena cava (SVC), right atrium (RA) and from there into the coronary sinus (CS). Alternatively, the access path may start in the femoral vein and through the inferior vena cava (IVC) into the heart. Other access routes may also be used, and each typically utilizes a percutaneous incision through which the guidewire and catheter are inserted into the vasculature, normally through a sealed introducer, and from there the physician controls the distal ends of the devices from outside the body.

FIG. 2 depicts one approach method for deploying an expandable shunt, wherein a guidewire 10 is introduced through the subclavian or jugular vein, through the superior vena cava (SVC) and into the coronary sinus. Once the guidewire provides a path, an introducer sheath (not shown) may be routed along the guidewire and into the patient's vasculature, typically with the use of a dilator. FIG. 2 shows a deployment catheter 12 extending from the SVC to the coronary sinus of the heart, the deployment catheter 12 having been passed through the introducer sheath which provides a hemostatic valve to prevent blood loss.

In some embodiments, the deployment catheter 12 may be about 30 cm long, and the guidewire 10 may be somewhat longer for ease of use. In certain embodiments, the deployment catheter 12 may function to form and to prepare an opening in the wall of the left atrium, and a separate placement or delivery catheter may be used for delivery of an expandable shunt. In various embodiments, the deployment catheter may be used for puncture preparation and may function as a shunt placement catheter with full functionality as well. In the present application, the terms "deployment catheter" and "delivery catheter" can be used to represent a catheter or introducer that provides one or both of these functions.

Since the coronary sinus is largely contiguous around the left atrium, there are a variety of possible acceptable placements for a stent. The site selected for placement of the stent may be made in an area where the tissue of the particular patient is less thick or less dense, as determined beforehand by non-invasive diagnostic means, such as a CT scan or radiographic technique, such as fluoroscopy or intravascular coronary echo (IVUS).

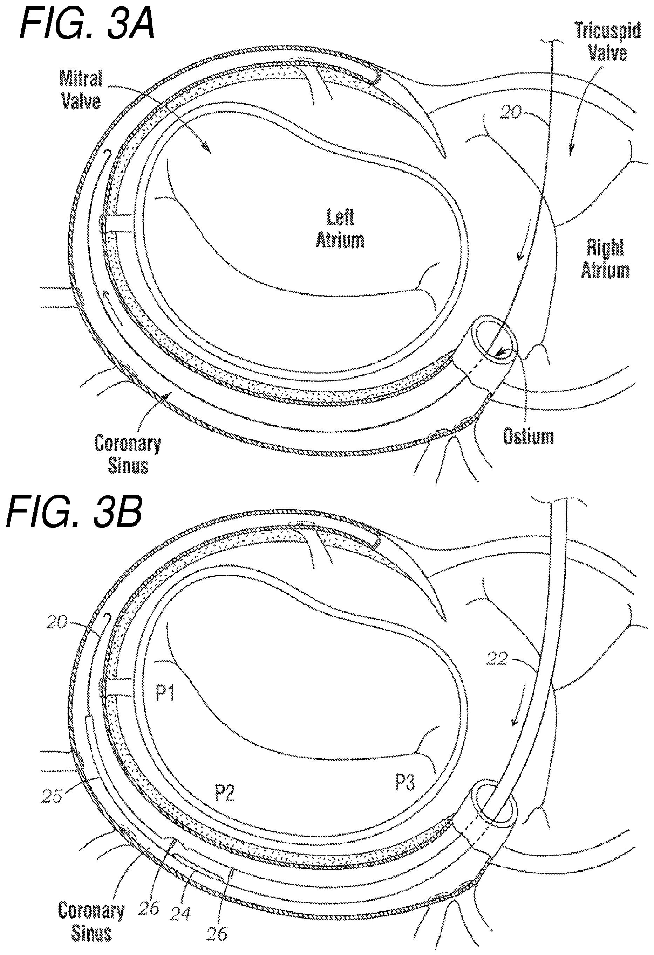

FIGS. 3A-3D illustrate schematic views of steps in making a puncture hole through a wall of the coronary sinus, as seen looking down on a section of the heart with the posterior aspect down. Initially, FIG. 3A shows a guidewire 20 being advanced from the right atrium into the coronary sinus through its ostium or opening. A puncture catheter 22 is then advanced over the guidewire 20, as seen in FIG. 3B. The puncture catheter 22 is introduced into the body through a proximal end of an introducer sheath (not shown). As is customary, an introducer sheath provides access to the particular vascular pathway (e.g., jugular or subclavian vein) and may have a hemostatic valve therein. While holding the introducer sheath at a fixed location, the surgeon manipulates the puncture catheter 22 to the implant site.

In some embodiments, a distal end of the puncture catheter 22 has a slight curvature built therein, with a radially inner and a radially outer side, to conform to the curvature of the coronary sinus. A securing mechanism 24 is exposed along a radially outer side of the catheter 22 adjacent a distal segment 25 that may be thinner than or tapered narrower from the proximal extent of the catheter 22. Radiopaque markers 26 on the catheter 22 can be used to help the surgeon determine the precise advancement distance for desired placement of the securing mechanism 24 within the coronary sinus. Desirably, the radiopaque markers 26 are C-shape bands that flank the proximal and distal ends of the securing mechanism 24.

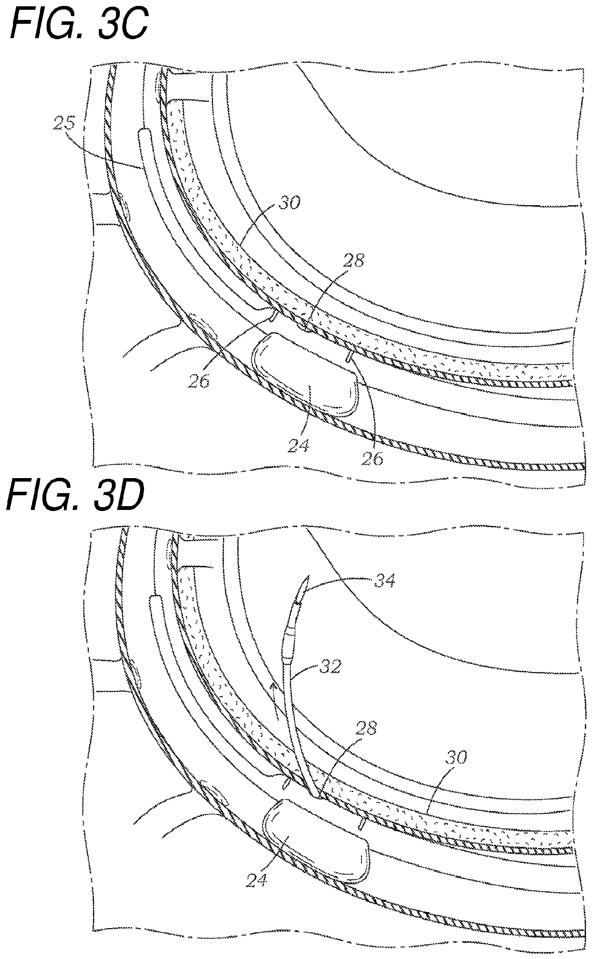

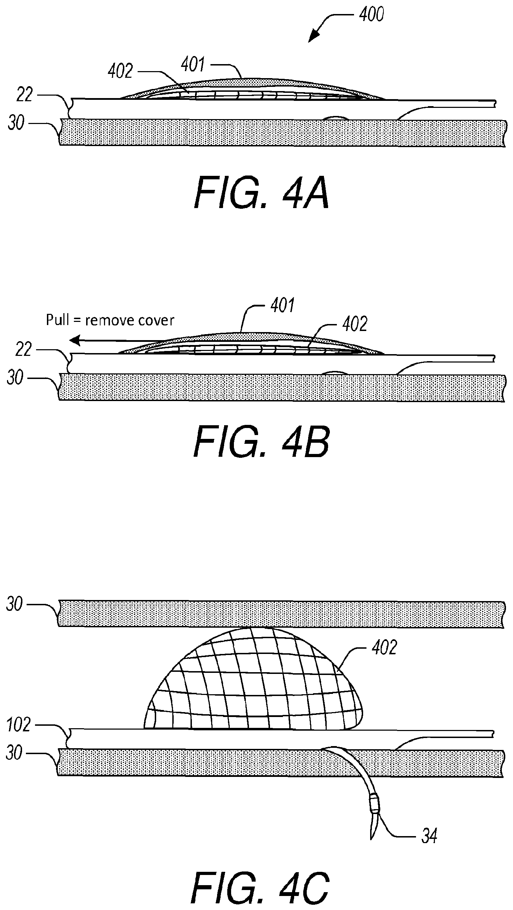

FIG. 3C shows outward deployment of the securing mechanism 24, which is to be considered a generic structure that is replaced by any of the securing mechanisms disclosed herein and described with reference to FIGS. 4A-7C. Deployment of the securing mechanism 24 presses the radially inner curve of the catheter against the luminal wall of the coronary sinus to provide wall apposition. Again, the securing mechanism 24 is located adjacent the distal segment 25 of the puncture catheter 22. In some embodiments, the securing mechanism 24 extends opposite a needle port 28 formed in the radially inner side wall of the catheter 22. Consequently, the needle port 28 abuts the luminal wall and faces toward a tissue wall 30 between the coronary sinus and the left atrium. Preferably, guided by visualizing the radiopaque markers 26, the surgeon advances the catheter 22 so that the needle port 28 is located within about 2-4 cm into the coronary ostia. This places the subsequent puncture approximately above the "P2" portion of the posterior leaflet of the mitral valve (when looking at the inflow side of the valve the posterior leaflet has P1-P2-P3 cusps in a CCW direction, as seen in FIG. 3B). The securing mechanism 24 may be centered diametrically across the catheter 22 from the needle port 28, or as shown may be offset in a proximal direction from the needle port 28 to improve leverage, or it may be offset in a distal direction from the needle port 28.

The curvature at the distal end of the puncture catheter 22 aligns to and "hugs" the anatomy within the coronary sinus and orients the needle port 28 inward, while the securing mechanism 24 holds the catheter 22 in place relative to the coronary sinus. Subsequently, as seen in FIG. 3D, a puncture sheath 32 having a puncture needle 34 with a sharp tip advances along the catheter 22 such that it exits the needle port 28 at an angle from the longitudinal direction of the catheter 22 and punctures through the wall 30 into the left atrium. The puncture sheath 32 has a built-in curvature at the end that "aligns" with the curvature of the anatomy ensuring that the needle 34 is oriented inward toward the left atrium. The securing mechanism 24 provides rigidity to the system and holds the needle port 28 against the wall 30 (e.g., the securing mechanism provides wall apposition). Preferably, the puncture needle 34 has a flattened configuration to form a linear incision and is mounted on the distal end of an elongated wire or flexible rod (not shown) that passes through a lumen of the puncture sheath 32.

### Securing Mechanisms

Described herein are mechanisms for securing a catheter within a vessel to facilitate puncturing a wall of the vessel with a needle that extends from the catheter. The securing mechanisms described herein can be implemented in a catheter, such as the catheter 22 described herein with respect to FIGS. 3A-3D. Additionally, the securing mechanisms described herein to provide the functionality of the generic securing mechanism 24 described herein with respect to FIGS. 3A-3D. In other words, the securing mechanisms described below can be used in place of the securing mechanism 24 described with respect to FIGS. 3A-3D.

The disclosed securing mechanisms are variations of the securing mechanism 24 that is used to secure the guidewire delivery catheter in place to enable puncturing the wall between the coronary sinus and the left atrium. In particular, the embodiments represent an improvement over a saline-filled anchor balloon because there is no risk of balloon burst and there is potential for better catheter securement. For balloon implementations, if the balloon bursts before the needle is deployed, the needle could deploy into the wrong space potentially causing severe damage to the patient. Moreover, a ruptured balloon could result in an embolism in the patient. The disclosed embodiments eliminate this risk because they do not employ saline-inflated balloons or other elements that require inflation using a liquid or gas. Furthermore, the disclosed embodiments eliminate the need to deflate the balloon, thereby providing a more streamlined procedure.

The disclosed securing mechanisms can include bubbles, meshes, flaps, or similar features that expand when activated to press against the wall of the coronary sinus. This force causes the distal end of the guidewire delivery catheter to be anchored to allow a needle to puncture the wall of the coronary sinus. Activation of the disclosed securing mechanisms can occur through removal of a restraint (e.g., a cover) or by pushing or pulling a wire. The disclosed securing mechanism materials can be mesh made from a stretchy, compliant wire material and/or a shape memory or self-expanding material. An example material can be a nickel titanium alloy such as Nitinol, but other materials may be used as well. The securing mechanisms activated by removal of a restraint may self-expand or may expand due to a spring within a mesh structure. The securing mechanisms activated by pushing or pulling a wire expand due to movement of the wire itself or due to movement of a component attached to the wire and to the anchor member.

The disclosed securing mechanisms are configured for use with a catheter, such as a guidewire delivery catheter as described herein. Also disclosed herein are guidewire delivery catheters that have a securing mechanism (or anchor member) that includes an activation member and an expanding member activated by the activation member. The securing mechanisms can be located on a side of the catheter that is opposite a needle port or similar feature to provide wall apposition when puncturing through the vessel wall.

A first example securing mechanism includes a cover that is removed to allow a self-expanding bubble to expand out of a side of the catheter. A second example securing mechanism includes a cover that is removed to allow a spring-loaded bubble to expand out of a side of the catheter. A third example securing mechanism includes a wire that is pushed to bunch up and to expand a mesh structure out of a side of the catheter. A fourth example securing mechanism has an expanding member that includes a fixed proximal structure and a movable distal structure with strips attached to the proximal and distal structures wherein pulling a wire causes the distal structure to move towards the proximal structure causing the strips to bulge outwards. The expanding members of the disclosed securing mechanisms are configured to be activated without the use of a liquid or gas as a means of inflation. That is, the disclosed securing mechanisms are not activated through inflation using liquid or gas.

FIGS. 4A-4C illustrate a first example securing mechanism 400. The first example securing mechanism 400 includes a self-expanding bubble 402 that is restrained by a cover 401 or other similar restraint, as shown in FIG. 4A. The securing mechanism 400 can be guided into place as part of the catheter 22, e.g., where the needle 34 is to puncture the wall of the coronary sinus 30. The bubble 402 is restrained during delivery using a restraining structure such as the cover 401. Once in place, the cover 401 can be removed, as shown in FIG. 4B. Removal of the cover 401 allows the self-expanding bubble structure 402 to expand out of the catheter 22, thereby securing the distal end of the guidewire delivery catheter 22 in place against the wall 30, as shown in FIG. 4C. In the expanded or deployed state, the bubble 402 provides wall apposition against the coronary sinus wall 30 for the needle 34 to puncture the opposite coronary sinus wall 30.

In some embodiments, the bubble 402 can be within the catheter 22. In some embodiments, the cover 401 can be part of an outer wall of the catheter 22. In such embodiments, the securing mechanism 400 is housed within the catheter 22 during delivery. In certain embodiments, the bubble 402 and the cover 401 are attached to an outer surface of the catheter 22. In various embodiments, the cover 401 is external to the catheter 22 and the bubble 402 is at least partially housed within the catheter 22.

The self-expanding bubble 402 can be made of a bunched material like a stretchy, compliant wire material. The material can be Nitinol, for example. The self-expanding bubble 402 can be a mesh material that allows fluids to flow through it, as opposed to a balloon that contains fluids (e.g., saline) that are used to inflate it. Beneficially, this allows fluid to flow through the vessel during the puncturing procedure.

The self-expanding bubble 402 can be shape-set such that, when expanded, the self-expanding bubble 402 creates a dome shape. In some embodiments, the self-expanding bubble 402 is configured to expand rapidly once the cover 401 is removed. The self-expanding bubble 402 can be retracted back into the catheter 22 using a pull wire or hypotube that pulls the self-expanding bubble 402 into the catheter 22. Once the self-expanding bubble is retracted, the cover 401 can be re-advanced to cover the self-expanding bubble 402. The cover 401 advantageously maintains the self-expanding bubble 402 within a contained area, prohibits the self-expanding bubble 402 from deploying prior to cover removal, and reduces the risk of damage during insertion.

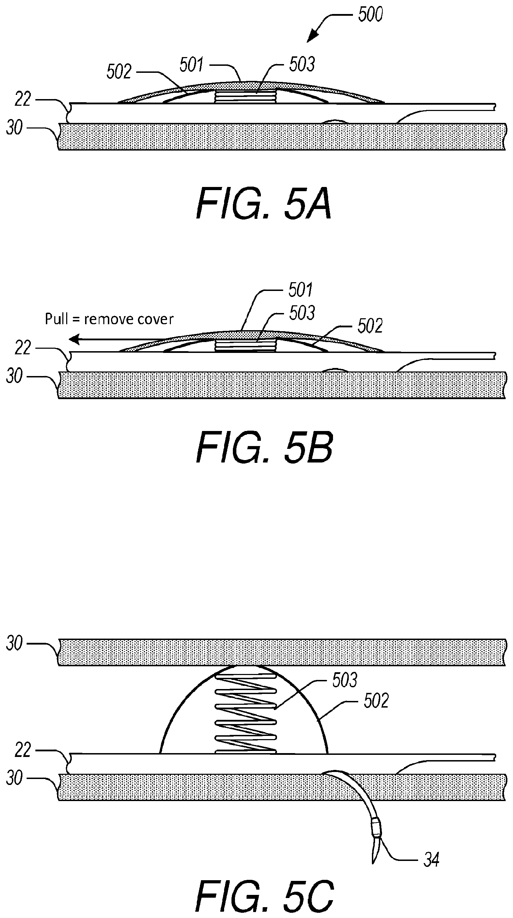

FIGS. 5A-5C illustrate a second example securing mechanism 500. The second example securing mechanism 500 includes a spring-loaded bubble 502 that expands when a restraint 501 is removed, expansion caused by a spring 503 encompassed within the bubble 502, as shown in FIG. 5A. The securing mechanism 500 can be guided into place as part of the catheter 22, e.g., where the needle 34 is to puncture the wall of the coronary sinus 30. Once in place, the restraint 501 can be removed, as shown in FIG. 5B. Removal of the restraint 501 allows the compressed spring 503 within the bubble 502 to decompress and to expand the mesh bubble 502 out of the catheter 22, thereby securing the distal end of the guidewire delivery catheter 22 in place against the wall 30, as shown in FIG. 5C. In the expanded or deployed state, the bubble 502 provides wall apposition against the coronary sinus wall 30 for the needle 34 to puncture the opposite coronary sinus wall 30. The bubble 502 can be a mesh material such as a stretchy, compliant wire material. Beneficially, this allows fluid to flow through the vessel during the puncturing procedure.

In some embodiments, the bubble 502 can be within the catheter 22. In some embodiments, the restraint 501 can be part of an outer wall of the catheter 22. In such embodiments, the securing mechanism 500 is housed within the catheter 22 during delivery. In certain embodiments, the bubble 502 and the restraint 501 are attached to an outer surface of the catheter 22. In various embodiments, the restraint 501 is external to the catheter 22 and the bubble 502 is at least partially housed within the catheter 22.

The spring-loaded bubble 502 is initially covered during delivery, the restraint 501 configured to keep the spring 503 in a compressed state. Thus, the restraint 501 is sufficiently resilient to maintain the spring 503 in the compressed state. The restraint 501 can be coupled to a pull wire or hypotube or other similar mechanism to withdraw the restraint 501 when the catheter 22 is in place at a desired location. Once the restraint 501 is removed, the spring 503 expands, thereby expanding the mesh or bubble 502. The bubble 502 forms a dome-like shape when expanded by the spring 503, rather than a cylinder-like shape conforming more closely to the spring 503. Thus, the material of the bubble 502 is sufficiently rigid to form the targeted dome-like shape while also being sufficiently compliant to be compressed by the restraint 501.

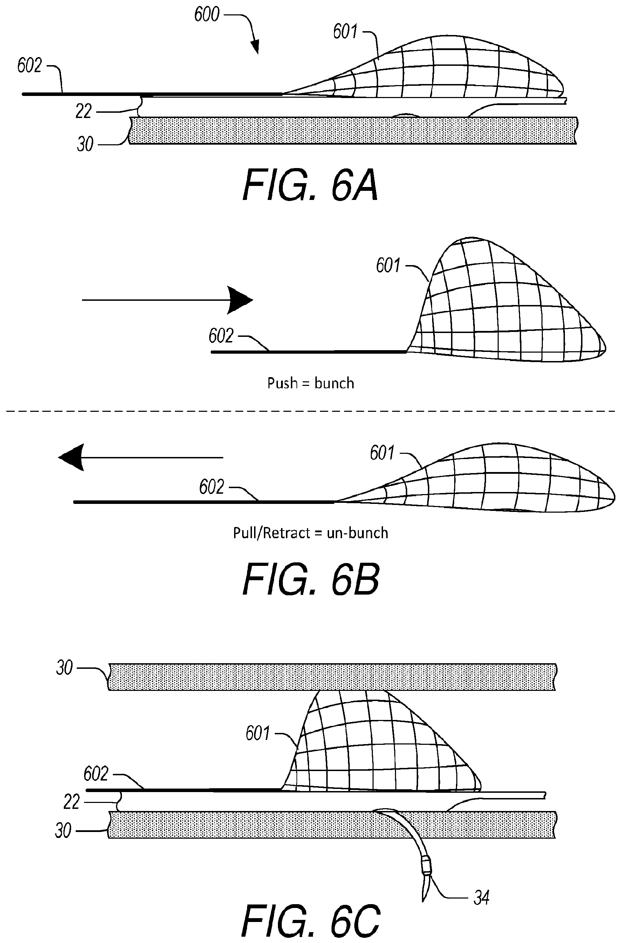

FIGS. 6A-6C illustrate a third example securing mechanism 600. The third example securing mechanism 600 includes a mesh structure 601 that has a proximal end attached to a wire 602, as shown in FIG. 6A. The securing mechanism 600 can be guided into place as part of the catheter 22, i.e., where the needle 34 is to puncture the wall 30 of the coronary sinus. Once in place, the wire 602 can be pushed to expand the mesh structure 601, as shown in FIG. 6B. Similarly, the mesh structure 601 can be retracted by pulling the wire 602, as shown in FIG. 6B. Pushing the wire 602 causes the mesh structure 601 to bunch up and to expand out of the catheter 22, thereby securing the distal end of the guidewire delivery catheter 22 in place against the wall 30 of the coronary sinus, as shown in FIG. 6C. This provides wall apposition for the needle 34 to puncture the wall 30 of the coronary sinus. The mesh structure 601 can be a mesh material such as a stretchy, compliant wire material. Beneficially, this allows fluid to flow through the vessel during the puncturing procedure. A distal portion of the mesh structure 601 can be attached to the catheter 22. This can be done to facilitate bunching of the mesh structure 601 as the wire 602 is advanced (e.g., pushed distally). In some embodiments, the mesh structure 601 is housed partially or entirely within the catheter 22 during delivery. In certain embodiments, the mesh structure 601 forms a part of an outer wall of the catheter 22.

In some embodiments, the mesh structure 601 comprises a fabric covering a wire that is advanced and that forms a dome shape. The fabric, in such embodiments, is expanded by the wire, which in turn is contained within the fabric thereby affecting the shape of the wire. This interplay between the fabric cover and the wire causes the dome shape to form, which presses against the coronary sinus wall 30 to provide apposition for the needle 34.

The wire 602 can extend from the proximal end of the mesh structure 601 to a proximal end of the catheter 22. In some embodiments, the wire 602 can be attached to the mesh structure 601 using a ring around the wire 601. In such embodiments, the ring has sliding capabilities. In some embodiments, the wire 601 is a hypotube that can be advanced and retracted independent of the guidewire of the catheter 22. Thus, when the catheter 22 is in place, the hypotube can be pushed to expand the mesh structure 601 to facilitate puncturing the wall 30, and after puncturing the wall 30, the hypotube can be pulled to retract the mesh structure 601.

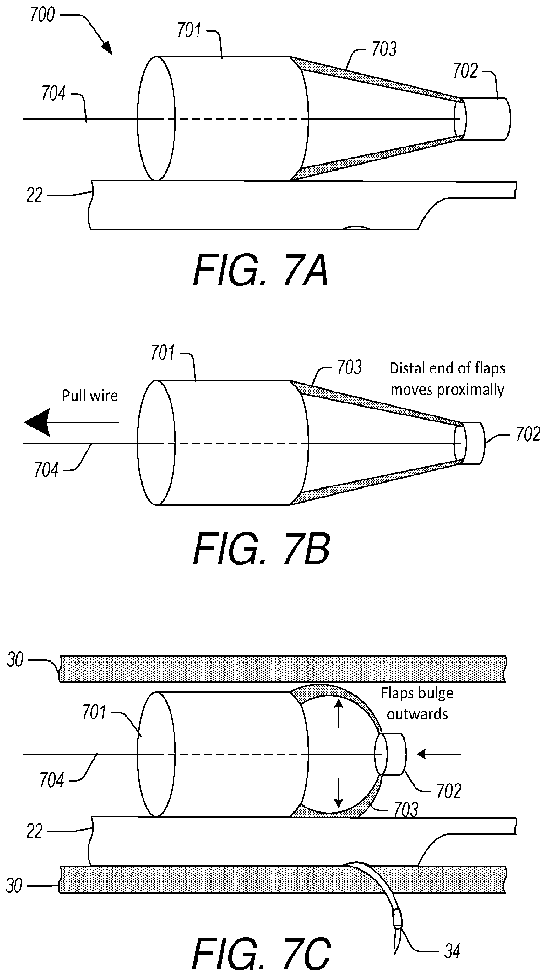

FIGS. 7A-7C illustrate a fourth example securing mechanism 700. The fourth example securing mechanism 700 includes include flaps, strips, or a wall (continuous or disjointed) 703 having proximal ends attached to a fixed, proximal structure 701 (e.g., a cylinder) and having distal ends attached to a movable, distal structure 702 (e.g., a cylinder) with a wire 704 that passes through the fixed, proximal structure 701 and attaches to the movable, distal structure 702, as shown in FIG. 7A. The securing mechanism 700 can be guided into place as part of the catheter 22, i.e., where the needle 34 is to puncture the wall 30 of the coronary sinus. Once in place, the wire 704 can be pulled. Pulling the wire 704 causes the distal structure 702 to move proximally toward the fixed, proximal structure 701, as shown in FIG. 7B. This relative movement causes the flaps or wall 703 to bulge outwards away from the catheter 22, thereby securing the distal end of the guidewire delivery catheter 22 in place against the wall 30 of the coronary sinus, as shown in FIG. 7C. The flaps or wall 703 can be any suitable material, such as a stretchy, compliant wire material. In some embodiments, the wire 704 runs through a hypotube, the wire 704 and hypotube surrounded by the proximal structure 701 and the wall 703.

Each of the securing mechanisms 400, 500, 600, and 700 can include marker bands that are radio opaque to indicate a position of the securing mechanism, as visualized using fluoroscopy. In such embodiments, the marker bands can be opposite the needle 34 to ensure that the needle is located in a desirable position prior to extending the needle to puncture the wall 30.

### Additional Embodiments

Depending on the embodiment, certain acts, events, or functions of any of the processes or algorithms described herein can be performed in a different sequence, may be added, merged, or left out altogether. Thus, in certain embodiments, not all described acts or events are necessary for the practice of the processes.

Conditional language used herein, such as, among others, "can," "could," "might," "may," "e.g.," and the like, unless specifically stated otherwise, or otherwise understood within the context as used, is intended in its ordinary sense and is generally intended to convey that certain embodiments include, while other embodiments do not include, certain features, elements and/or steps. Thus, such conditional language is not generally intended to imply that features, elements and/or steps are in any way required for one or more embodiments or that one or more embodiments necessarily include logic for deciding, with or without author input or prompting, whether these features, elements and/or steps are included or are to be performed in any particular embodiment. The terms "comprising," "including," "having," and the like are synonymous, are used in their ordinary sense, and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Conjunctive language such as the phrase "at least one of X, Y and Z," unless specifically stated otherwise, is understood with the context as used in general to convey that an item, term, element, etc. may be either X, Y or Z. Thus, such conjunctive language is not generally intended to imply that certain embodiments require at least one of X, at least one of Y and at least one of Z to each be present.

It should be appreciated that in the above description of embodiments, various features are sometimes grouped together in a single embodiment, Figure, or description thereof for the purpose of streamlining the disclosure and aiding in the understanding of one or more of the various inventive aspects. This method of disclosure, however, is not to be interpreted as reflecting an intention that any claim require more features than are expressly recited in that claim. Moreover, any components, features, or steps illustrated and/or described in a particular embodiment herein can be applied to or used with any other embodiment(s). Further, no component, feature, step, or group of components, features, or steps are necessary or indispensable for each embodiment. Thus, it is intended that the scope of the inventions herein disclosed and claimed below should not be limited by the particular embodiments described above, but should be determined only by a fair reading of the claims that follow.

It should be understood that certain ordinal terms (e.g., "first" or "second") may be provided for ease of reference and do not necessarily imply physical characteristics or ordering. Therefore, as used herein, an ordinal term (e.g., "first," "second," "third," etc.) used to modify an element, such as a structure, a component, an operation, etc., does not necessarily indicate priority or order of the element with respect to any other element, but rather may generally distinguish the element from another element having a similar or identical name (but for use of the ordinal term). In addition, as used herein, indefinite articles ("a" and "an") may indicate "one or more" rather than "one." Further, an operation performed "based on" a condition or event may also be performed based on one or more other conditions or events not explicitly recited.

Unless otherwise defined, all terms (including technical and scientific terms) used herein have the same meaning as commonly understood by one of ordinary skill in the art to which example embodiments belong. It be further understood that terms, such as those defined in commonly used dictionaries, should be interpreted as having a meaning that is consistent with their meaning in the context of the relevant art and not be interpreted in an idealized or overly formal sense unless expressly so defined herein.

Although certain preferred embodiments and examples are disclosed below, inventive subject matter extends beyond the specifically disclosed embodiments to other alternative embodiments and/or uses and to modifications and equivalents thereof. Thus, the scope of the claims that may arise herefrom is not limited by any of the particular embodiments described below. For example, in any method or process disclosed herein, the acts or operations of the method or process may be performed in any suitable sequence and are not necessarily limited to any particular disclosed sequence. Various operations may be described as multiple discrete operations in turn, in a manner that may be helpful in understanding certain embodiments; however, the order of description should not be construed to imply that these operations are order dependent. Additionally, the structures, systems, and/or devices described herein may be embodied as integrated components or as separate components. For purposes of comparing various embodiments, certain aspects and advantages of these embodiments are described. Not necessarily all such aspects or advantages are achieved by any particular embodiment. Thus, for example, various embodiments may be carried out in a manner that achieves or optimizes one advantage or group of advantages as taught herein without necessarily achieving other aspects or advantages as may also be taught or suggested herein.

The spatially relative terms "outer," "inner," "upper," "lower," "below," "above," "vertical," "horizontal," and similar terms, may be used herein for ease of description to describe the relations between one element or component and another element or component as illustrated in the drawings. It be understood that the spatially relative terms are intended to encompass different orientations of the device in use or operation, in addition to the orientation depicted in the drawings. For example, in the case where a device shown in the drawing is turned over, the device positioned "below" or "beneath" another device may be placed "above" another device. Accordingly, the illustrative term "below" may include both the lower and upper positions. The device may also be oriented in the other direction, and thus the spatially relative terms may be interpreted differently depending on the orientations.

Unless otherwise expressly stated, comparative and/or quantitative terms, such as "less," "more," "greater," and the like, are intended to encompass the concepts of equality. For example, "less" can mean not only "less" in the strictest mathematical sense, but also, "less than or equal to."

Delivery systems as described herein may be used to position catheter tips and/or catheters to various areas of a human heart. For example, a catheter tip and/or catheter may be configured to pass from the right atrium into the coronary sinus. However, it will be understood that the description can refer or generally apply to positioning of catheter tips and/or catheters from a first body chamber or lumen into a second body chamber or lumen, where the catheter tips and/or catheters may be bent when positioned from the first body chamber or lumen into the second body chamber or lumen. A body chamber or lumen can refer to any one of a number of fluid channels, blood vessels, and/or organ chambers (e.g., heart chambers). Additionally, reference herein to "catheters," "tubes," "sheaths," "steerable sheaths," and/or "steerable catheters" can refer or apply generally to any type of elongate tubular delivery device comprising an inner lumen configured to slidably receive instrumentation, such as for positioning within an atrium or coronary sinus, including for example delivery catheters and/or cannulas. It will be understood that other types of medical implant devices and/or procedures can be delivered to the coronary sinus using a delivery system as described herein, including for example ablation procedures, drug delivery and/or placement of coronary sinus leads.
Examples are set out in the following list of numbered clauses:
1. A guidewire delivery catheter used to implant a shunt between the coronary sinus and the left atrium, the catheter including a securing mechanism that comprises:
   a removable cover coupled to a wire; and
   an expanding member activated by removal of the removable cover, the removable cover configured to be pulled by the wire to expose the expanding member causing the expanding member to expand out of the catheter to secure a distal end of the catheter in place in the coronary sinus.
2. The catheter of clause 1, wherein the expanding member comprises a mesh structure.
3. The catheter of any of clauses 1-2, wherein the expanding member comprises a compliant wire material.
4. The catheter of any of clauses 1-3, wherein the expanding member comprises a self-expanding bubble.
5. The catheter of any of clauses 1-4, wherein the expanding member comprises a spring-loaded bubble.
6. The catheter of clause 5, wherein the spring is retained in a compressed state by the cover and removal of the cover allows the spring to decompress, thereby expanding the spring-loaded bubble to form a dome shape.
7. The catheter of any of clauses 1-6, wherein the expanding member is configured to be retracted by re-advancing the removable cover over the expanding member.
8. The catheter of any of clauses 1-7, wherein activation of the expanding member causes the expanding member to press against a vessel wall to provide wall apposition for the catheter to facilitate puncturing the vessel wall.
9. The catheter of any of clauses 1-8, wherein the wire comprises a hypotube.
10. The catheter of any of clauses 1-9, wherein the expanding member is not activated through inflation using liquid or gas.
11. A guidewire delivery catheter used to implant a shunt between the coronary sinus and the left atrium, the catheter including a securing mechanism that comprises:
   an activation member including a wire; and
   an expanding member activated by the activation member, a proximal side of the expanding member coupled to a distal end of the activation member, the expanding member configured to expand out of the catheter responsive to the activation member being advanced distally, the expanding member thereby securing a distal end of the catheter in place in the coronary sinus.
12. The catheter of clause 11, wherein the expanding member comprises a mesh structure.
13. The catheter of any of clauses 11-12, wherein the expanding member comprises a compliant wire material.
14. The catheter of any of clauses 11-13, wherein the expanding member is configured to be retracted by pulling the activation member proximally.
15. The catheter of any of clauses 11-14, wherein a distal portion of the expanding member is fixed to the catheter.
16. The catheter of any of clauses 11-15, wherein activation of the expanding member causes the expanding member to press against a vessel wall to provide wall apposition for the catheter to facilitate puncturing the vessel wall.
17. The catheter of any of clauses 11-16, wherein the expanding member comprises a fabric covering that contains a distal portion of the activation member such that as the activation member is advanced distally, the distal portion of the activation member curves within the fabric covering to cause the fabric covering to form a dome shape.
18. The catheter of any of clauses 11-17, wherein the wire comprises a hypotube.
19. The catheter of any of clauses 11-18, wherein the expanding member is not activated through inflation using liquid or gas.
20. A guidewire delivery catheter used to implant a shunt between the coronary sinus and the left atrium, the catheter including a securing mechanism that comprises:
   an activation member including a wire coupled to a movable distal structure; and
   an expanding member activated by the activation member, the expanding member including a fixed proximal structure and the movable distal structure with pliable material forming a wall attached to the fixed proximal structure and to the movable distal structure such that pulling the activation member causes the movable distal structure to move towards the fixed proximal structure causing the wall to bulge outwards, thereby securing a distal end of the catheter in place in the coronary sinus.
21. The catheter of clause 20, wherein the wall comprises a compliant wire material.
22. The catheter of any of clauses 20-21, wherein the expanding member is configured to be retracted by pushing the activation member distally.
23. The catheter of any of clauses 20-22, wherein activation of the expanding member causes the expanding member to press against a vessel wall to provide wall apposition for the catheter to facilitate puncturing the vessel wall.
24. The catheter of any of clauses 20-23, wherein the activation member comprises a wire running through a hypotube, the hypotube surrounded by the fixed proximal structure.
25. The catheter of any of clauses 20-24, wherein the wire comprises a hypotube.
26. The catheter of any of clauses 20-25, wherein the expanding member is not activated through inflation using liquid or gas.

## Claims

1. A puncture catheter (22) for creating a puncture hole between the coronary sinus and the left atrium, the catheter (22) including a securing mechanism (24) that comprises:
a removable cover (401, 501); and
an expanding member (402, 502) activated by removal of the removable cover (401, 501), the removable cover (401, 501) configured to be removed to expose the expanding member (402, 502) causing the expanding member (402, 502) to expand out of the catheter (22) to secure a distal end of the catheter (22) in place in the coronary sinus; and
wherein activation of the expanding member causes the expanding member to press against a wall (30) of the coronary sinus to provide wall apposition for the catheter to facilitate puncturing the wall (30) of the coronary sinus.

2. The catheter of claim 1, wherein the expanding member (402, 502) comprises a mesh structure (601).

3. The catheter of any of claims 1-2, wherein the expanding member (402, 502) comprises a compliant wire material.

4. The catheter of any of claims 1-3, wherein the expanding member (402, 502) comprises a self-expanding bubble (402).

5. The catheter of any of claims 1-4, wherein the expanding member (402, 502) comprises a spring-loaded bubble (502).

6. The catheter of claim 5, wherein the spring (503) is retained in a compressed state by the cover (501) and removal of the cover (501) allows the spring (503) to decompress, thereby expanding the spring-loaded bubble (502) to form a dome shape.

7. The catheter of any of claims 1-6, wherein the expanding member (402, 502) is configured to be retracted by re-advancing the removable cover (401, 501) over the expanding member (402, 502).

8. The catheter of any of claims 1-7, wherein the removable cover (401, 501) is coupled to a wire and wherein the removable cover is configured to be pulled by the wire to expose the expanding member.

9. The catheter of claim 8, wherein the wire comprises a hypotube.

10. The catheter of any of claims 1-9, wherein the expanding member (402, 502) is not activated through inflation using liquid or gas.
